# EUROPEAN PATENT SPECIFICATION

(11) **EP 3 416 146 B1**
(45) Date of publication and mention of the grant of the patent: **01.01.2020**
(21) Application number: 17759170.8
(22) Date of filing: 22.02.2017
(51) Int. Cl.: G08B 21/04, A61B 5/00, A61B 7/04, A61B 5/01, A61B 5/021, G08B 25/10, G01N 33/497, A61B 5/11, A61B 5/024, A61B 5/0205, A61B 5/0402

(54) **HUMAN BODY CONDITION AND BEHAVIOUR MONITORING AND ALARM SYSTEM**
ÜBERWACHUNGS- UND ALARMSYSTEM FÜR DEN ZUSTAND UND DAS VERHALTEN DES MENSCHLICHEN KÖRPERS
SYSTÈME DE SURVEILLANCE ET D'ALARME D'ÉTAT ET DE COMPORTEMENT DE CORPS HUMAIN

(30) Priority: 02.03.2016 CN 201620159214 U
(43) Date of publication of application: 19.12.2018
(73) Proprietor: LEADER TIGER SOFTWARE (FUZHOU) CO., LTD., Fuzhou, Fujian 350000 (CN)
(72) Inventor: TANG, Minghong, Fuzhou, Fujian 350000 (CN)
(74) Representative: Gulde & Partner
(86) International application number: PCT/CN2017/074385
(87) International publication number: WO 2017/148319

(56) References cited:
- WO-A1-2016/005805
- CN-A- 102 068 261
- CN-A- 103 310 585
- CN-A- 103 330 554
- CN-A- 103 705 214
- CN-A- 103 908 233
- CN-A- 104 873 174
- CN-A- 105 118 236
- CN-A- 105 608 837
- CN-U- 205 582 205
- US-A1- 2011 245 633
- US-A1- 2012 218 301
- US-A1- 2012 290 266
- US-A1- 2016 035 205

## Description

### Technical Field

The invention relates to the field of smart wearable devices, in particular to a human body condition and behavior monitoring alarm system.

### Description of Related Art

With the advance of the social technology, more and more smart wearable devices have entered people's life. Modern people pay more and more attention to physical health, and following the current research and development tide of smart wear, more and more researchers put research and development emphasis into the field of smart wear for health. For instance, Chinese Invention Patent 201110371533. 7 titled 'Heart Rate Warning System' discloses a smart garment with a heart rate warning function, which can detect the psychological condition of the wearer in real time and send out a prompt alarm in time when the heart rate of the wearer is abnormal.

With the development of society, besides the requirement for heart rate monitoring, people also have the requirement for more comprehensively monitoring of various physical conditions and even behavior conditions, such as abnormal behaviors including excited emotions, physical conflicts and falling of bodies so as to know and handle these conditions as early as possible.

However, at present, only devices for monitoring physical conditions have been developed by those skilled in the field of smart wear, and smart wearable devices capable of monitoring behavior conditions of human bodies are not available yet. US2012218301A1 relates to an augmented reality eyepiece, associated control technologies, and applications for use.

### Technical Problems

The objective of the invention is to provide a human body condition and behavior monitoring alarm system, which is capable of monitoring both physical conditions and behavior conditions of human bodies.

### Solution to Problems

### Technical Solution

The invention is realized through the following technical scheme: a human body condition and behavior monitoring alarm system is characterized by:
comprising:
a master chip used for receiving, sending and processing information and sending alarm commands;
a display connected with the master chip and used for displaying information data;
a temperature and humidity sensor connected with the master chip and used for sensing and collecting human body temperature and humidity data in real time and transmitting the collected data to the master chip;
a blood pressure sensor connected with the master chip and used for sensing and collecting human body blood pressure data in real time and transmitting the collected data to the master chip;
a brain wave sensor connected with the master chip and used for sensing and collecting human body brain wave data in real time and transmitting the collected data to the master chip;
an electronic gyroscope connected with the master chip and used for sensing and collecting human body posture data in real time and transmitting the collected data to the master chip;
a heart rate sensor connected with the master chip and used for sensing and collecting human body heart rate data in real time and transmitting the collected data to the master chip;
a pressure sensor connected with the master chip and used for sensing and collecting human body weight data as well as pressure data of a garment a human body in real time and transmitting the collected data to the master chip;
a hydrogen sulfide sensor connected with the master chip and used for sensing and collecting concentration data of hydrogen sulfide discharged by the human body in real time and transmitting the collected data to the master chip;
a noise detection module connected with the master chip and used for monitoring the loudness of noise in the ambient environment;
a Bluetooth module connected with the master chip and used for sending data and alarm information; and
a power supply module connected with the master chip and used for providing a system power supply.

The master chip is further connected with a voice alarm module used for sending out an alarm signal. When the human body temperature and humidity data, the human body blood pressure data, the human body brain wave data, the human body heart rate data, the human body posture data and the hydrogen sulfide concentration data received by the master chip exceed set values, the master chip instantly instructs the voice alarm module to send out an alarm signal.

The master chip is further connected with a recording module used for recording sounds in the ambient environment. When detecting that the noise, sensed by the noise detection module, in the ambient environment is greater than a set value, the master chip instantly controls the recording module to record the noise. Furthermore, the human body condition and behavior monitoring alarm system further comprises an electronic stethoscope connected with the recording module and used for acquiring human body sound data.

The operating principle of the human body condition and behavior monitoring alarm system is as follows:

The human body condition and behavior monitoring alarm system of the invention detects the health condition of the human body in the following way:
All the sensors transmit the human body temperature and humidity data, the human body blood pressure data, the human body brain wave data, the human body posture data and other data to the master chip in real time, the master chip compares all the sets of data with human body pathological data indicators set and stored in the master chip and instantly instructs the voice alarm module to send out an alarm signal once finding that the data transmitted from the sensors are abnormal, and at this moment, the alarm information is sent to an external receiving terminal through a Bluetooth, WIFI or GSM module so that the wearer and relevant personnel can know an abnormal physical condition in time.

Through noise monitoring, when increased suddenly, surrounding noise is recorded and transmitted to relevant personnel, and the relevant personnel can know the current condition, such as quarrels and physical conflicts with other people, of the wearer by playing the record back.

The master chip can automatically figure out whether or not the posture of the wearer is normal by monitoring changes of the human body posture data in real time and instantly instructs the voice alarm module to send out an alarm signal once realizing an abnormal condition (such as sudden falling), and at this moment, the alarm information is sent to the external receiving terminal through a Bluetooth, WIFI and wireless communication module so that the wearer and relevant personnel can know the abnormal behavior condition in time.

Herein, the brain wave sensor is generally disposed in a cap; the hydrogen sulfide sensor is generally disposed on a pair of trousers and used for detecting whether or not the wearer relieve himself/herself so that relevant personnel can realize and handle the defecation condition in time; and two pressure sensors are adopted generally, one pressure sensor is disposed in a shoe sole and used for measuring the weight of the human body, the other pressure sensor is disposed in the shoulder part of the garment, and the master chip can figure out the thickness of the garment worn by the wearer according to the pressure sensed by the pressure sensor on the shoulder part of the garment and then automatically amplifies the volume of the speaker when the garment is thick.

To prevent the sensors from being wetted by water when the garment is washed, connection circuits of the sensors are all embedded in an interlayer of the garment, and the joints between the connection circuits and the master chip are of a waterproof structure.

In order to better implement the scheme, the following optimization scheme is further provided:

Furthermore, the human body condition and behavior monitoring alarm system further comprises a GSM module connected with the master chip and used for sending alarm information to a mobile phone.

The GSM module is provided with an SIM card holder, a GPRS module and a GPS positioning module.

Furthermore, the human body condition and behavior monitoring alarm system further comprises a WIFI module connected with the master chip and used for sending alarm information to an external routing device.

Furthermore, the human body condition and behavior monitoring alarm system further comprises a touch screen connected with the master chip and used for operating the system.

The human body sound data acquired by the electronic stethoscope are transmitted to the recording module so as to be recorded.

A sound hole of the electronic stethoscope is generally disposed at a position, close to the chest, of the inner side of the garment so that abnormal human body sound data of the wearer can be found in time.

Furthermore, the master chip is an STM32 master chip.

Furthermore, the display is an OLED display screen.

Furthermore, the temperature and humidity sensor, the blood pressure sensor, the brain wave sensor, the electronic gyroscope, the heart rate sensor, the pressure sensor and the hydrogen sulfide sensor are all sleeved with waterproof shells.

Furthermore, the human body condition and behavior monitoring alarm system further comprises a camera connected with the master chip and used for recording images of the ambient environment.

The monitor can know the condition around the wearer through the images recorded by the camera, and the camera is generally disposed at a position corresponding to the chest or on a neckline.

Furthermore, the noise detection module is provided with a microphone.

The microphone is used for acquiring sounds around the wearer and can be disposed in the garment, on the neckline or at any position corresponding to the chest.

Furthermore, the voice alarm module is provided with a speaker.

The speaker can be used for playing voice alarm information and also can be used for playing key sounds or other sounds in the system.

Furthermore, the human body condition and behavior monitoring alarm system further comprises a solar cell connected with the master chip and used for providing a system power supply.

Furthermore, the human body condition and behavior monitoring alarm system further comprises a radio module 23 connected with the master chip 1.

### Beneficial Effects of the Invention

### Beneficial Effects

Compared with the prior art, the invention has the following beneficial effect:

First, through comparison of the data collected by various sensors of the invention, abnormal body conditions of the wearer can be realized in time, and alarm information can be sent out in time.

Second, through the means including sound recording, video recording, photographing, posture detection and the like, the behavior condition of the wearer can be well monitored, and in-time intervention can be conducted when an abnormal behavior occurs.

Third, the human body condition and behavior monitoring alarm system of the invention is convenient to wear, has rich functions and is suitable for users at different ages.

### Brief Description of Drawings

### Description of Drawings

FIG. 1 is a block diagram of modules of the invention;
FIG. 2 is a use diagram of the invention.

### Description of Reference Signs:

1-master chip, 2-display, 3-temperature and humidity sensor, 4-blood pressure sensor, 5-brain wave sensor, 6-electronic gyroscope, 7-noise detection module, 8-recording module, 9-voice alarm module, 10-Bluetooth module, 11-power supply module, 12-GSM module, 13-WIFI module, 14-touch screen, 15-heart rate sensor, 16-pressure sensor, 17-hydrogen sulfide sensor, 18-electronic stethoscope, 19-camera, 20-microphone, 21-speaker, 22-solar cell, 23-radio module.

### Embodiments of the Invention

### Detailed Description of the Invention

A detailed description of the invention is given with the accompanying drawings as follows:

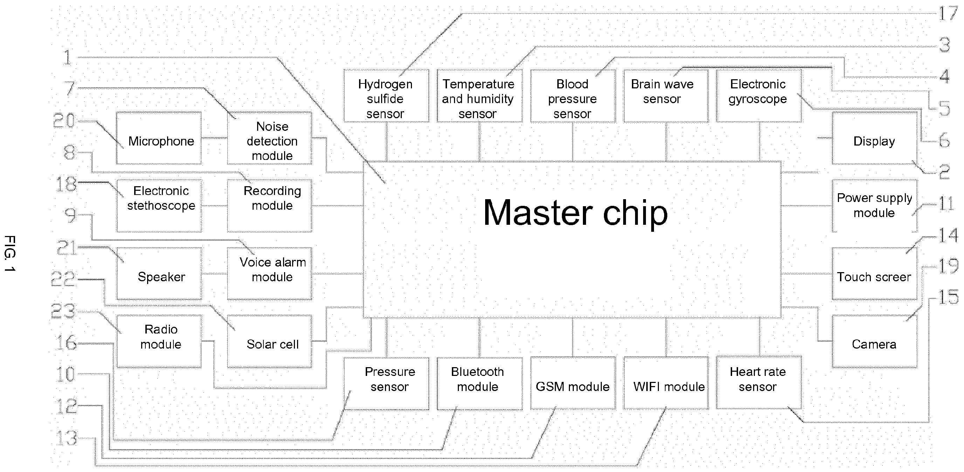

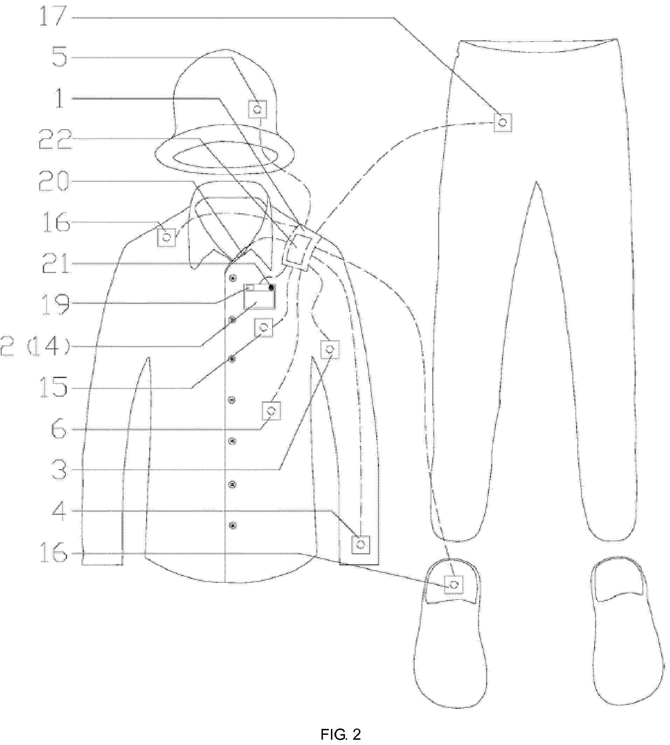

As is shown in FIGs. 1-2, a human body condition and behavior monitoring alarm system of the invention comprises:
a master chip 1 used for receiving, sending and processing information and sending alarm commands;
a display 2 connected with the master chip 1 and used for displaying information data;
a temperature and humidity sensor 3 connected with the master chip 1 and used for sensing and collecting human body temperature and humidity data in real time and transmitting the collected data to the master chip 1;
a blood pressure sensor 4 connected with the master chip 1 and used for sensing and collecting human body blood pressure data in real time and transmitting the collected data to the master chip 1;
a brain wave sensor 5 connected with the master chip 1 and used for sensing and collecting human body brain wave data in real time and transmitting the collected data to the master chip 1;
an electronic gyroscope 6 connected with the master chip 1 and used for sensing and collecting human body posture data in real time and transmitting the collected data to the master chip 1;
a heart rate sensor 15 connected with the master chip 1 and used for sensing and collecting human body heart rate data in real time and transmitting the collected data to the master chip 1;
a pressure sensor 16 connected with the master chip 1 and used for sensing and collecting human body weight data as well as pressure data of a garment to a human body in real time and transmitting the collected data to the master chip 1;
a hydrogen sulfide sensor 17 connected with the master chip 1 and used for sensing and collecting concentration data of hydrogen sulfide discharged by the human body in real time and transmitting the collected data to the master chip 1;
a noise detection module 7 connected with the master chip 1 and used for monitoring the loudness of noise in the ambient environment;
a Bluetooth module 10 connected with the master chip 1 and used for sending data and alarm information; and
a power supply module 11 connected with the master chip 1 and used for providing a system power supply.

The master chip 1 is further connected with a voice alarm module 9 used for sending out an alarm signal. When the human body temperature and humidity data, the human body blood pressure data, the human body brain wave data, the human body heart rate data, the human body posture data and the hydrogen sulfide concentration data received by the master chip 1 exceed set values, the master chip 1 instantly instructs the voice alarm module 9 to send out an alarm signal.

The master chip 1 is further connected with a recording module 8 used for recording sounds in the ambient environment. When detecting that the noise, sensed by the noise detection module 7, in the ambient environment is greater than a set value, the master chip 1 instantly controls the recording module 8 to record the noise.

In order to better implement the scheme, the following optimization scheme is further provided:

Furthermore, the human body condition and behavior monitoring alarm system further comprises a GSM module 12 connected with the master chip 1 and used for sending alarm information to a mobile phone.

The GSM module 12 is provided with an SIM card holder, a GPRS module and a GPS positioning module.

Furthermore, the human body condition and behavior monitoring alarm system further comprises a WIFI module 13 connected with the master chip 1 and used for sending alarm information to an external routing device.

Furthermore, the human body condition and behavior monitoring alarm system further comprises a touch screen 14 connected with the master chip 1 and used for displaying the system time and operating the system.

Furthermore, the human body condition and behavior monitoring alarm system further comprises an electronic stethoscope 18 connected with the recording module 8 and used for acquiring human body sound data.

The human body sound data acquired by the electronic stethoscope 18 are transmitted to the recording module 8 so as to be recorded.

Furthermore, the display 2 is an OLED display screen.

Furthermore, the master chip 1 is an STM32 master chip.

Furthermore, the temperature and humidity sensor 3, the blood pressure sensor 4, the brain wave sensor 5, the electronic gyroscope 6, the heart rate sensor 15, the pressure sensor 16 and the hydrogen sulfide sensor 17 are all sleeved with waterproof shells.

Furthermore, the human body condition and behavior monitoring alarm system further comprises a camera 19 connected with the master chip 1 and used for recording images of the ambient environment.

Furthermore, the noise detection module 7 is provided with a microphone 20.

Furthermore, the voice alarm module 9 is provided with a speaker 21.

Furthermore, the human body condition and behavior monitoring alarm system further comprises a solar cell 22 connected with the master chip 1 and used for providing a system power supply.

Furthermore, the human body condition and behavior monitoring alarm system further comprises a radio module 23 connected with the master chip 1.

Although the invention is illustrated and explained through the specific embodiment and substitutes of the specific embodiment, it would appreciate that various modifications and changes made without deviating from the scope of the invention can also be implemented. Thus, the invention is not limited in any sensor except by the claims and equivalent conditions of the claims.

## Claims

1. A human body condition and behavior monitoring alarm system, comprising:
a master chip (1) used for receiving, sending and processing information and sending alarm commands;
a display (2) connected with the master chip (1) and used for displaying information data;
a temperature and humidity sensor (3) connected with the master chip (1) and used for sensing and collecting human body temperature and humidity data in real time and transmitting the collected data to the master chip (1);
a blood pressure sensor (4) connected with the master chip (1) and used for sensing and collecting human body blood pressure data in real time and transmitting the collected data to the master chip (1);
a brain wave sensor (5) connected with the master chip (1) and used for sensing and collecting human body brain wave data in real time and transmitting the collected data to the master chip (1);
an electronic gyroscope (6) connected with the master chip (1) and used for sensing and collecting human body posture data in real time and transmitting the collected data to the master chip (1);
a heart rate sensor (15) connected with the master chip (1) and used for sensing and collecting human body heart rate data in real time and transmitting the collected data to the master chip (1);
a pressure sensor (16) connected with the master chip (1) and used for sensing and collecting human body weight data as well as pressure data of a garment to a human body in real time and transmitting the collected data to the master chip (1); a hydrogen sulfide sensor (17) connected with the master chip (1) and used for sensing and collecting concentration data of hydrogen sulfide discharged by the human body in real time and transmitting the collected data to the master chip (1); a noise detection module (7) connected with the master chip (1) and used for monitoring a loudness of noise in an ambient environment;
a Bluetooth module (10) connected with the master chip (1) and used for sending data and alarm information; and
a power supply module (11) connected with the master chip (1) and used for providing a system power supply;
wherein, the master chip (1) is further connected with a voice alarm module (9) used for sending out an alarm signal, and when the human body temperature and humidity data, the human body blood pressure data, the human body brain wave data, the human body heart rate data, the human body posture data and the hydrogen sulfide concentration data received by the master chip (1) exceed set values, the master chip (1) instantly instructs the voice alarm module (9) to send out an alarm signal;
the master chip (1) is further connected with a recording module (8) used for recording sounds in the ambient environment; and when detecting that noise sensed by the noise detection module (7) in the ambient environment is greater than a set value, the master chip (1) instantly controls the recording module (8) to record the noise,
**characterized in that** the human body condition and behavior monitoring alarm system further comprises an electronic stethoscope (18) connected with the recording module (8) and used for acquiring human body sound data.

2. The human body condition and behavior monitoring alarm system according to Claim 1, wherein further comprising a GSM module (12) connected with the master chip (1) and used for sending alarm information to a mobile phone, wherein, the GSM module (12) is provided with an SIM card holder, a GPRS module and a GPS positioning module.

3. The human body condition and behavior monitoring alarm system according to Claim 1, wherein further comprising a WIFI module (13) connected with the master chip (1) and used for sending alarm information to an external routing device.

4. The human body condition and behavior monitoring alarm system according to Claim 1, wherein further comprising a touch screen (14) connected with the master chip (1) and used for operating the system.

5. The human body condition and behavior monitoring alarm system according to Claim 1, wherein, the human body sound data acquired by the electronic stethoscope (18) are transmitted to the recording module (8) so as to be recorded.

6. The human body condition and behavior monitoring alarm system according to Claim 1, wherein the display (2) is an OLED display screen.

7. The human body condition and behavior monitoring alarm system according to Claim 1, wherein the temperature and humidity sensor (3), the blood pressure sensor (4), the brain wave sensor (5), the electronic gyroscope (6), the heart rate sensor (15), the pressure sensor (16) and the hydrogen sulfide sensor (17) are all sleeved with waterproof shells.

8. The human body condition and behavior monitoring alarm system according to Claim 1, wherein further comprising a camera (19) connected with the master chip (1) and used for recording images of the ambient environment, wherein the noise detection module (7) is provided with a microphone (20), and the voice alarm module (9) is provided with a speaker (21).

9. The human body condition and behavior monitoring alarm system according to Claim 1, wherein further comprising a radio module (23) connected with the master chip (1).

10. The human body condition and behavior monitoring alarm system according to Claim 1, wherein further comprising a solar cell (22) connected with the master chip (1) and used for providing a system power supply.

## Patentansprüche

1. Ein Überwachungs- und Alarmsystem für den Zustand und das Verhalten des menschlichen Körpers, aufweisend:
einen Master-Chip (1), der verwendet wird, um Informationen zu empfangen, zu senden und zu verarbeiten und Alarmbefehle zu senden;
eine Anzeige (2), die mit dem Master-Chip (1) verbunden ist und verwendet wird, um Informationsdaten anzuzeigen;
einen Temperatur- und Feuchtigkeitssensor (3), der mit dem Master-Chip (1) verbunden ist und verwendet wird, um Temperatur- und Feuchtigkeitsdaten des menschlichen Körpers in Echtzeit zu erfassen und zu sammeln und die gesammelten Daten zum Master-Chip (1) zu übertragen;
einen Blutdrucksensor (4), der mit dem Master-Chip (1) verbunden ist und verwendet wird, um Blutdruckdaten des menschlichen Körpers in Echtzeit zu erfassen und zu sammeln und die gesammelten Daten zum Master-Chip (1) zu übertragen;
einen Gehirnwellensensor (5), der mit dem Master-Chip (1) verbunden ist und verwendet wird, um Gehirnwellendaten des menschlichen Körpers in Echtzeit zu erfassen und zu sammeln und die gesammelten Daten zum Master-Chip (1) zu übertragen;
ein elektronisches Gyroskop (6), das mit dem Master-Chip (1) verbunden ist und verwendet wird, um Haltungsdaten des menschlichen Körpers in Echtzeit zu erfassen und zu sammeln und die gesammelten Daten zum Master-Chip (1) zu übertragen;
einen Herzfrequenzsensor (15), der mit dem Master-Chip (1) verbunden ist und verwendet wird, um Herzfrequenzdaten des menschlichen Körpers in Echtzeit zu erfassen und zu sammeln und die gesammelten Daten zum Master-Chip (1) zu übertragen;
einen Drucksensor (16), der mit dem Master-Chip (1) verbunden ist und verwendet wird, um Gewichtsdaten des menschlichen Körpers sowie Druckdaten eines Kleidungsstücks an einem menschlichen Körper in Echtzeit zu erfassen und zu sammeln und die gesammelten Daten zum Master-Chip (1) zu übertragen;
einen Schwefelwasserstoffsensor (17), der mit dem Master-Chip (1) verbunden ist und verwendet wird, um Daten über die Konzentration von Schwefelwasserstoff, der vom menschlichen Körper abgegeben wird, in Echtzeit zu erfassen und zu sammeln und die gesammelten Daten zum Master-Chip (1) zu übertragen;
ein Geräuscherfassungsmodul (7), das mit dem Master-Chip (1) verbunden ist und verwendet wird, um eine Geräuschstärke in einer Außenumgebung zu überwachen;
ein Bluetooth-Modul (10), das mit dem Master-Chip (1) verbunden ist und verwendet wird, um Daten und Alarminformationen zu senden; und
ein Energieversorgungsmodul (11), das mit dem Master-Chip (1) verbunden ist und verwendet wird, um eine Systemenergieversorgung bereitzustellen;
wobei der Master-Chip (1) ferner mit einem Sprachalarmmodul (9), das verwendet wird, um ein Alarmsignal auszusenden, verbunden ist, und der Master-Chip (1), wenn die Temperatur- und Feuchtigkeitsdaten des menschlichen Körpers, die Blutdruckdaten des menschlichen Körpers, die Gehirnwellendaten des menschlichen Körpers, die Herzfrequenzdaten des menschlichen Körpers, die Haltungsdaten des menschlichen Körpers und die Schwefelwasserstoffkonzentrationsdaten, die vom Master-Chip (1) empfangen werden, Sollwerte überschreiten, unverzüglich das Sprachalarmmodul (9) anweist, ein Alarmsignal auszusenden;
wobei der Master-Chip (1) ferner mit einem Aufzeichnungsmodul (8), das verwendet wird, um Geräusche in der Außenumgebung aufzuzeichnen, verbunden ist, und der Master-Chip (1), wenn erfasst wird, dass ein vom Geräuscherfassungsmodul (7) in der Außenumgebung erfasstes Geräusch stärker ist als ein Sollwert, unverzüglich das Aufzeichnungsmodul (8) steuert, so dass es das Geräusch aufzeichnet,
**dadurch gekennzeichnet, dass** das Überwachungs- und Alarmsystem für den Zustand und das Verhalten des menschlichen Körpers ferner ein elektronisches Stethoskop (18), das mit dem Aufzeichnungsmodul (8) verbunden ist und verwendet wird, um Geräuschdaten des menschlichen Körpers zu erfassen, aufweist.

2. Das Überwachungs- und Alarmsystem für den Zustand und das Verhalten des menschlichen Körpers nach Anspruch 1, ferner aufweisend ein GSM-Modul (12), das mit dem Master-Chip (1) verbunden ist und verwendet wird, um Alarminformationen zu einem Mobiltelefon zu senden,
wobei das GSM-Modul (12) mit einem SIM-Kartenhalter, einem GPRS-Modul und einem GPS-Ortungsmodul bereitgestellt wird.

3. Das Überwachungs- und Alarmsystem für den Zustand und das Verhalten des menschlichen Körpers nach Anspruch 1, ferner aufweisend ein WIFI-Modul (13), das mit dem Master-Chip (1) verbunden ist und verwendet wird, um Alarminformationen zu einer externen Routing-Vorrichtung zu senden.

4. Das Überwachungs- und Alarmsystem für den Zustand und das Verhalten des menschlichen Körpers nach Anspruch 1, ferner aufweisend einen Berührungsbildschirm (14), der mit dem Master-Chip (1) verbunden ist und verwendet wird, um das System zu betreiben.

5. Das Überwachungs- und Alarmsystem für den Zustand und das Verhalten des menschlichen Körpers nach Anspruch 1, wobei die Geräuschdaten des menschlichen Körpers, die vom elektronischen Stethoskop (18) erfasst werden, zum Aufzeichnungsmodul (8) übertragen werden, um aufgezeichnet zu werden.

6. Das Überwachungs- und Alarmsystem für den Zustand und das Verhalten des menschlichen Körpers nach Anspruch 1, wobei die Anzeige (2) ein OLED-Anzeigebildschirm ist.

7. Das Überwachungs- und Alarmsystem für den Zustand und das Verhalten des menschlichen Körpers nach Anspruch 1, wobei der Temperatur- und Feuchtigkeitssensor (3), der Blutdrucksensor (4), der Gehirnwellensensor (5), das elektronische Gyroskop (6), der Herzfrequenzsensor (15), der Drucksensor (16) und der Schwefelwasserstoffsensor (17) alle mit wasserdichten Schalen umhüllt sind.

8. Das Überwachungs- und Alarmsystem für den Zustand und das Verhalten des menschlichen Körpers nach Anspruch 1, ferner aufweisend eine Kamera (19), die mit dem Master-Chip (1) verbunden ist und verwendet wird, um Bilder der Außenumgebung aufzuzeichnen, wobei das Geräuscherfassungsmodul (7) mit einem Mikrophon (20) bereitgestellt wird und das Sprachalarmmodul (9) mit einem Lautsprecher (21) bereitgestellt wird.

9. Das Überwachungs- und Alarmsystem für den Zustand und das Verhalten des menschlichen Körpers nach Anspruch 1, ferner aufweisend ein Funkmodul (23), das mit dem Master-Chip (1) verbunden ist.

10. Das Überwachungs- und Alarmsystem für den Zustand und das Verhalten des menschlichen Körpers nach Anspruch 1, ferner aufweisend eine Solarzelle (22), die mit dem Master-Chip (1) verbunden ist und verwendet wird, um eine Systemenergieversorgung bereitzustellen.

## Revendications

1. Un système de surveillance et d'alarme d'état et de comportement de corps humain, comprenant :
une puce maître (1) utilisée pour recevoir, envoyer et traiter des informations et envoyer des commandes d'alarme ;
un affichage (2) connecté à la puce maître (1) et utilisé pour afficher des données d'information ;
un capteur de température et d'humidité (3) connecté à la puce maître (1) et utilisé pour détecter et collecter des données de température et d'humidité du corps humain en temps réel et transmettre les données collectées à la puce maître (1) ;
un capteur de pression artérielle (4) connecté à la puce maître (1) et utilisé pour détecter et collecter des données de pression artérielle du corps humain en temps réel et transmettre les données collectées à la puce maître (1) ;
un capteur d'ondes cérébrales (5) connecté à la puce maître (1) et utilisé pour détecter et collecter des données d'ondes cérébrales du corps humain en temps réel et transmettre les données collectées à la puce maître (1) ;
un gyroscope électronique (6) connecté à la puce maître (1) et utilisé pour détecter et collecter des données de posture du corps humain en temps réel et transmettre les données collectées à la puce maître (1) ;
un capteur de fréquence cardiaque (15) connecté à la puce maître (1) et utilisé pour détecter et collecter des données de fréquence cardiaque du corps humain en temps réel et transmettre les données collectées à la puce maître (1) ;
un capteur de pression (16) connecté à la puce maître (1) et utilisé pour détecter et collecter des données de poids du corps humain ainsi que des données de pression d'un vêtement sur un corps humain en temps réel et transmettre les données collectées à la puce maître (1) ;
un capteur de sulfure d'hydrogène (17) connecté à la puce maître (1) et utilisé pour détecter et collecter des données de concentration de sulfure d'hydrogène déchargé par le corps humain en temps réel et transmettre les données collectées à la puce maître (1) ;
un module de détection de bruit (7) connecté à la puce maître (1) et utilisé pour surveiller une intensité de bruit dans un environnement ambiant ;
un module Bluetooth (10) connecté à la puce maître (1) et utilisé pour envoyer des données et des informations d'alarme ; et
un module d'alimentation en énergie (11) connecté à la puce maître (1) et utilisé pour fournir une alimentation en énergie du système ;
la puce maître (1) étant en outre connectée à un module d'alarme vocale (9) utilisé pour envoyer un signal d'alarme, et, lorsque les données de température et d'humidité du corps humain, les données de pression artérielle du corps humain, les données d'ondes cérébrales du corps humain, les données de fréquence cardiaque du corps humain, les données de posture du corps humain, et les données de concentration de sulfure d'hydrogène reçues par la puce maître (1) dépassent des valeurs de consigne, la puce maître (1) ordonnant instantanément au module d'alarme vocale (9) d'envoyer un signal d'alarme ;
la puce maître (1) étant en outre connectée à un module d'enregistrement (8) utilisé pour enregistrer des sons dans l'environnement ambiant, et, lors de la détection que le bruit détecté par le module de détection de bruit (7) dans l'environnement ambiant est supérieur à une valeur de consigne, la puce maître (1) commandant instantanément le module d'enregistrement (8) pour qu'il enregistre le bruit ;
**caractérisé en ce que** le système de surveillance et d'alarme d'état et de comportement de corps humain comprend en outre un stéthoscope électronique (18) connecté au module d'enregistrement (8) et utilisé pour acquérir des données de sons du corps humain.

2. Le système de surveillance et d'alarme d'état et de comportement de corps humain selon la revendication 1, comprenant en outre un module GSM (12) connecté à la puce maître (1) et utilisé pour envoyer des informations d'alarme à un téléphone mobile,
le module GSM (12) étant pourvu d'un support de carte SIM, d'un module GPRS et d'un module de positionnement GPS.

3. Le système de surveillance et d'alarme d'état et de comportement de corps humain selon la revendication 1, comprenant en outre un module Wi-Fi (13) connecté à la puce maître (1) et utilisé pour envoyer des informations d'alarme à un dispositif de routage externe.

4. Le système de surveillance et d'alarme d'état et de comportement de corps humain selon la revendication 1, comprenant en outre un écran tactile (14) connecté à la puce maître (1) et utilisé pour faire fonctionner le système.

5. Le système de surveillance et d'alarme d'état et de comportement de corps humain selon la revendication 1, dans lequel les données de sons du corps humain acquises par le stéthoscope électronique (18) sont transmises au module d'enregistrement (8) pour être enregistrées.

6. Le système de surveillance et d'alarme d'état et de comportement de corps humain selon la revendication 1, dans lequel l'affichage (2) est un écran d'affichage OLED.

7. Le système de surveillance et d'alarme d'état et de comportement de corps humain selon la revendication 1, dans lequel le capteur de température et d'humidité (3), le capteur de pression artérielle (4), le capteur d'ondes cérébrales (5), le gyroscope électronique (6), le capteur de fréquence cardiaque (15), le capteur de pression (16) et le capteur de sulfure d'hydrogène (17) sont tous enveloppés de coquilles imperméables à l'eau.

8. Le système de surveillance et d'alarme d'état et de comportement de corps humain selon la revendication 1, comprenant en outre une caméra (19) connectée à la puce maître (1) et utilisée pour enregistrer des images de l'environnement ambiant, le module de détection de bruit (7) étant pourvu d'un microphone (20), et le module d'alarme vocale (9) étant pourvu d'un haut-parleur (21).

9. Le système de surveillance et d'alarme d'état et de comportement de corps humain selon la revendication 1, comprenant en outre un module radio (23) connecté à la puce maître (1).

10. Le système de surveillance et d'alarme d'état et de comportement de corps humain selon la revendication 1, comprenant en outre une cellule solaire (22) connectée à la puce maître (1) et utilisée pour fournir une alimentation en énergie du système.
